# EUROPEAN PATENT APPLICATION

(11) **EP 4 597 108 A1**
(43) Date of publication of application: **06.08.2025**
(21) Application number: 23871931.4
(22) Date of filing: 13.09.2023
(51) Int. Cl.: G01N 33/543

(54) **IMMUNOCHROMATOGRAPHIC CARTRIDGE**

(30) Priority: 26.09.2022 JP 2022153102
(71) Applicant: FUJIFILM Corporation, Tokyo 106-8620 (JP)
(72) Inventor: USHIKURA, Shinichi, Ashigarakami-gun, Kanagawa 258-8538 (JP)
(74) Representative: Dehns Germany Partnerschaft mbB
(86) International application number: PCT/JP2023/033374
(87) International publication number: WO 2024/070698

(57) **Abstract**

An immunochromatographic cartridge includes an assay strip having an assay region in which the sample is developed and a test substance contained in the sample is captured, a binding substance that specifically binds to the test substance and is labeled with a luminescent protein, a luminescent substrate solution holding part that accommodates a luminescent substrate solution containing a luminescent substrate that is supplied to the assay region and that reacts with the luminescent protein to generate light, and a case that accommodates the assay strip and the luminescent substrate solution holding part and that includes a photometric window part through which light from the assay region is able to be measured from the outside, in which the case further includes a retention part that retains the luminescent substrate solution on the assay region in a case where the luminescent substrate solution is developed on the assay strip.

## Description

### BACKGROUND OF THE INVENTION

### 1. Field of the Invention

The present disclosure relates to an immunochromatographic cartridge.

### 2. Description of the Related Art

An immunological measuring method is widely used as a method of qualitatively or quantitatively measuring a test substance present in a biological specimen such as urine or blood. Among the above, an immunochromatographic method is highly convenient since the operation is easy and the measurement can be carried out in a short time.

In the immunochromatographic method, an assay strip, on which an antibody (a capture antibody) that specifically binds to a test substance (for example, an antigen) is immobilized in an assay region, is used. As an assay method used in the immunochromatographic method, an antibody subjected to labeling (a labeled antibody), which specifically binds to an antigen, and a sample are developed on an assay strip to form a composite body of the capture antibody-antigen-labeled antibody in the assay region in a case where the antigen is contained in the sample. Then, a signal such as color development due to the labeling of the labeled antibody is detected to qualitatively or quantitatively measure the antigen which is a test substance.

In such an immunochromatographic method, to avoid a problem that a test substance is not detected and shows a false negative due to low sensitivity even though the test substance is contained, a technique for improving sensitivity has been proposed. As one of the techniques for improving sensitivity, WO2016/114122A and WO2017/104143A disclose a technique for amplifying a signal by attaching silver to a metal colloid label.

In a case where a signal amplification technique using silver amplification, which is disclosed in WO2016/114122A and WO2017/104143A, is used, it is possible to perform a highly sensitive detection. For this silver amplification, it is necessary to supply two types of amplification liquids, that is, a liquid that catalyzes amplification, such as a solution containing a silver ion reducing agent, and a liquid that performs amplification, such as a solution containing a silver ion, to the immunochromatographic carrier. JP2016-114122A and WO2017-104143A propose a cartridge that includes two types of reagents such as a solution containing an assay strip and a silver ion reducing agent, and a solution containing a silver ion, and that can efficiently supply the two types of reagents to the assay strip.

On the other hand, JP2015-105858A discloses a chemiluminescence immunochromatographic method as another method of improving sensitivity, in which a luminescent protein is used as a label, and light generated by a chemical reaction of the luminescent protein with a luminescent substrate is detected. In the chemiluminescence immunochromatographic method, a sample and a labeled antibody labeled with a luminescent protein are developed on an assay strip on which a capture antibody is immobilized in an assay region. Then, a luminescent substrate, which reacts with the luminescent protein as a label to generate light, is developed on the assay strip. The luminescence, which occurs by the reaction between the luminescent protein captured through the labeled antibody captured in the assay region and the luminescent substrate, is detected to qualitatively or quantitatively measure the antigen which is a test substance. JP2015-105858A proposes a method in which weir bodies is disposed to surround an assay region, and then a solution containing a luminescent substrate is put on the assay region to sufficiently permeate the luminescent substrate into the assay region.

In addition, JP2018-522221A discloses a method for detecting fluorescence generated from a fluorescent substance by using bioluminescence resonance energy transfer (BRET) that occurs between a luminescent protein and a fluorescent substance, which is a chemiluminescence immunochromatographic method. According to the chemiluminescence immunochromatographic method using BRET, since fluorescence is generated only in a region where the luminescent protein and the fluorescent substance are close to each other, the background can be reduced and the sensitivity can be further increased.

### SUMMARY OF THE INVENTION

By using the weir body disclosed in JP2015-105858A, the luminescent substrate can be sufficiently permeated into the assay strip by retaining the luminescent substrate on the assay region. However, according to the measuring method of JP2015-105858A, in a case where the luminescent substrate is put on the membrane, the user needs to dispose the weir bodies such that the weir bodies surround the assay region of the membrane and put the solution containing the luminescent substrate in the region surrounded by the weir bodies. Therefore, the burden on the user in the assay is large, and the assay takes time.

A practical cartridge that can more easily perform the chemiluminescence immunochromatographic method as disclosed in JP2015-105858A and JP2018-522221A has not been developed so far.

An object of the present disclosure is to provide an immunochromatographic cartridge that can reliably and easily realize an assay by a chemiluminescence immunochromatographic method.

An immunochromatographic cartridge of the present disclosure comprising an assay strip having an assay region in which a sample is developed and a test substance contained in the sample is captured, a binding substance that specifically binds to the test substance and is labeled with a luminescent protein, a luminescent substrate solution holding part that accommodates a luminescent substrate solution containing a luminescent substrate that is supplied to the assay region and that reacts with the luminescent protein to generate light, and a case that accommodates the assay strip and the luminescent substrate solution holding part and that includes a photometric window part through which light from the assay region is measurable from outside, in which the case further includes a retention part that retains the luminescent substrate solution on the assay region in a case where the luminescent substrate solution is developed on the assay strip.

In the case, the luminescent substrate solution holding part is disposed to be capable of supplying the luminescent substrate solution to the assay strip from above the assay strip, and the case may include a flow channel forming part that is disposed to face a surface of the assay strip in the assay region, a part of the flow channel forming part constitutes the photometric window part, and the luminescent substrate solution may be supplied to the assay region through between the assay strip and a facing surface of the flow channel forming part facing the assay strip as a flow channel.

In the flow channel, in a case where the assay region is set as a reference position, a side of a supply position where the luminescent substrate solution is supplied is set as an upstream side, and an opposite side is set as a downstream side, the immunochromatographic cartridge may have two dam parts that are provided at two positions of the upstream side and the downstream side of the flow channel and that dam a flow of the luminescent substrate solution supplied to the flow channel from the supply position, two side walls that are connected to the two dam parts and that are disposed on a lateral of the flow channel, the two dam parts and the two side walls are connected to the facing surface, and the retention part is constituted by the two dam parts, the two side walls, and the facing surface, and may define a space surrounding a periphery of the assay region.

The facing surface of the flow channel may have a first region including a facing region facing the assay region and a second region that surrounds a periphery of the first region and that has an affinity for the luminescent substrate solution lower than that of the first region, and the retention part may be constituted by the first region and the second region.

The first region may be a hydrophilized region subjected to a hydrophilization treatment, and the second region may be a hydrophobized region subjected to a hydrophobization treatment.

At least a part of the luminescent substrate solution holding part is constituted by a film, the case further includes a perforating member that is disposed to face the film of the luminescent substrate solution holding part and that perforates the film, and in the case, the film may be perforated by the perforating member by relative movement of the luminescent substrate solution holding part and the perforating member, to supply the luminescent substrate solution onto the assay strip.

The case may include a variable part that is deformable inward in a portion covering the luminescent substrate solution holding part, and that moves the luminescent substrate solution holding part toward the perforating member by applying an external force.

The case includes a hole portion in a portion covering the luminescent substrate solution holding part, and the luminescent substrate solution holding part may be movable toward the perforating member by a pressing member to be inserted into the hole portion.

The luminescent substrate solution holding part is preferably constituted by a light shielding member.

In the case, a washing solution holding part that accommodates a washing solution which is supplied to the assay region and by which a binding substance non-specifically adsorbed to the assay region is washed is preferably further provided.

The assay strip includes a binding substance for capturing that is immobilized on the assay region, that is labeled with a fluorescent substance, and that specifically binds to the test substance, and the assay strip includes a labeled binding substance that is disposed on an upstream side of the assay region in a case of developing the sample and that is supplied to the assay region, as the binding substance, and in a case where the luminescent substrate is supplied to the assay region in a state where the labeled binding substance is captured by the binding substance for capturing that is immobilized on the assay region, through the test substance, a bioluminescence resonance energy transfer may occur in which energy of light generated by a reaction between the luminescent protein and the luminescent substrate is transferred to the fluorescent substance to generate fluorescence.

The assay strip includes the binding substance as a binding substance for capturing that is immobilized in the assay region, and the assay strip further includes a labeled binding substance that is disposed on an upstream side of the assay region in a case where the sample is developed, that specifically binds to the test substance, that is labeled with a fluorescent substance, and that is supplied to the assay region, and in a case where the luminescent substrate is supplied to the assay region in a state where the labeled binding substance is captured by the binding substance for capturing that is immobilized in the assay region, through the test substance, a bioluminescence resonance energy transfer may occur in which energy of light generated by a reaction between the luminescent protein and the luminescent substrate is transferred to the fluorescent substance to generate fluorescence.

The case preferably includes an optical filter that attenuates light generated by the reaction between the luminescent substrate solution and the luminescent protein and that allows transmission of the fluorescence through the photometric window part.

According to the cartridge of the present disclosure, an assay by a chemiluminescence immunochromatographic method can be reliably and easily realized.

### BRIEF DESCRIPTION OF THE DRAWINGS

Fig. 1 is a perspective view of a cartridge.
Fig. 2 is an exploded perspective view of the cartridge.
Fig. 3 is a broken side view of the cartridge.
Fig. 4 is a broken side view of the cartridge, which shows a state where a first pressing operation part is pushed in.
Fig. 5 is a broken side view of the cartridge, which shows a state where in addition to the first pressing operation part, a second pressing operation part is pushed in.
Fig. 6 is a perspective view of an intermediate case member.
Fig. 7 is a perspective view showing a back surface side of the intermediate case member.
Fig. 8 is an explanatory view of a retention part, in which Fig. 8A is a partially broken side view of the cartridge, Fig. 8B is a B-B cross-sectional view in Fig. 8A, and Fig. 8C is a C-C cross-sectional view in Fig. 8A.
Fig. 9 is a diagram showing a procedure of an immunochromatographic assay (1).
Fig. 10 is a diagram showing a procedure of an immunochromatographic assay (2).
Fig. 11 is an explanatory view of light from an assay region L1.
Fig. 12 is a view showing a back surface side of an intermediate case member of a modification example.
Fig. 13 is a partially broken side view of the cartridge provided with a retention part of a modification example.
Fig. 14 is a partially broken side view and a plan view of a cartridge of a modification example.
Fig. 15 is a view showing a state in which a luminescent substrate solution holding part is moved in the cartridge of Fig. 14.
Fig. 16 is a perspective view of an intermediate case member provided with an optical filter in a photometric window part.
Fig. 17 is a partially broken side view of the cartridge provided with the intermediate case member shown in Fig. 16.
Fig. 18 is an explanatory view of light from an assay region L1 of another aspect.
Fig. 19 is an explanatory view of light from an assay region L1 in a chemiluminescence immunochromatographic method not using BRET.

### DESCRIPTION OF THE PREFERRED EMBODIMENTS

Hereinafter, specific embodiments of the immunochromatographic cartridge according to the present disclosure will be described with reference to the drawings. The constituent elements indicated by the same reference numerals in the drawings mean the same constituent elements.

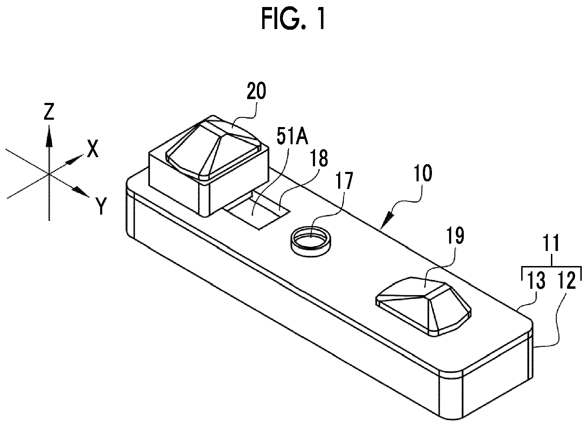

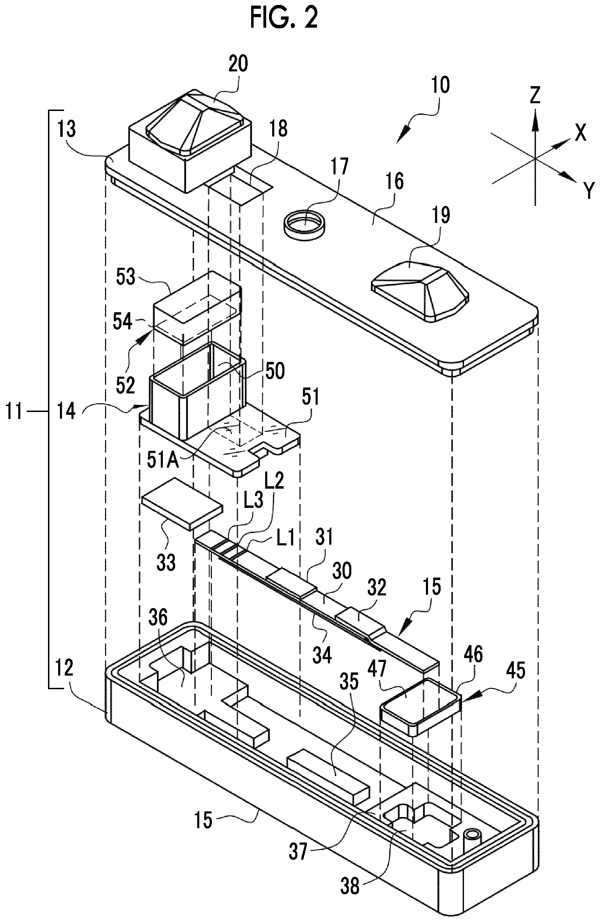

Fig. 1 is a perspective view showing an appearance of an immunochromatographic cartridge 10 (hereinafter, referred to as a cartridge 10) according to one embodiment, and Fig. 2 is an exploded perspective view of the cartridge 10.

As shown in Figs. 1 and 2, the cartridge 10 accommodates an assay strip 15 in a case 11. The cartridge 10 is a single-use type that is used one by one for each sample, and is an assay tool for assaying the presence or absence of a test substance in a sample by using an immune reaction.

The case 11 is constituted by a case main body 12, a cover member 13, and an intermediate case member 14 (see Fig. 2). The case main body 12 is a box having an accommodation space that is surrounded by a bottom plate having an elongated rectangular plate shape and four side plates standing vertically from four sides of the bottom plate. An assay strip 15 having the assay region L1 is accommodated in the accommodation space of the case main body 12. The intermediate case member 14 is disposed between the case main body 12 and the cover member 13, and includes a photometric window part 51A that is exposed to the outside.

It is noted that both the X direction and the Y direction in Figs. 1 and 2 are directions along the horizontal plane and are orthogonal to each other. The X direction is a direction along a short side of the case 11 and the Y direction is a direction along a long side of the case 11. The Z direction is a direction along the vertical direction and is orthogonal to the X direction and the Y direction. The same applies to the following drawings.

A sample dropping port 17 and a photometry opening 18 are formed in the cover member 13. In addition, a first pressing operation part 19 and a second pressing operation part 20 are provided in the cover member 13. The sample dropping port 17, the photometry opening 18, the first pressing operation part 19, and the second pressing operation part 20 are integrally formed.

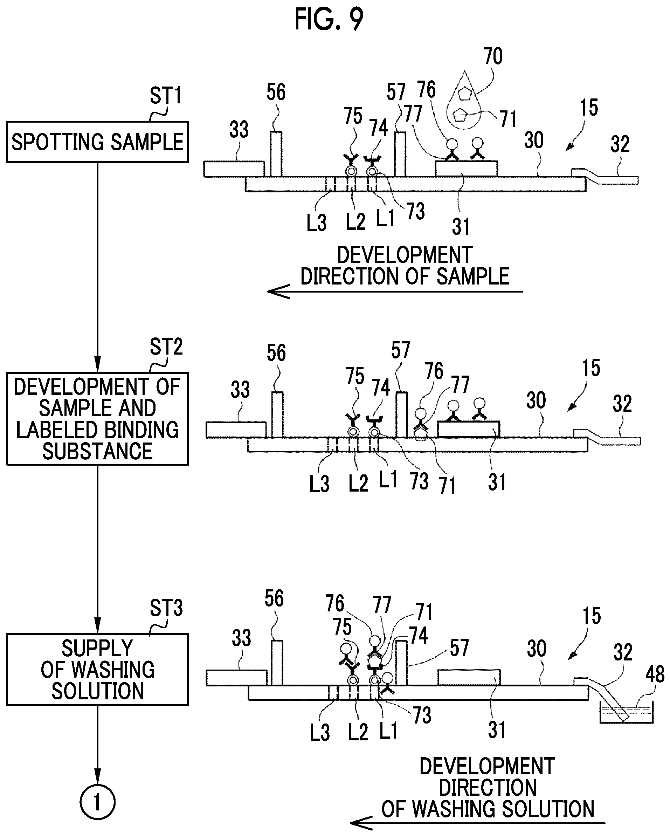

The sample dropping port 17 is a round hole to which a sample solution 70 (see Fig. 9) containing a sample is added dropwise, and has a boss shape in which an edge protrudes from the surface. The sample dropping port 17 is formed in a central part of the cover member 13. The sample may be any sample as long as it can contain a test substance 71, and it is not particularly limited. The sample includes a biological specimen of an individual as a target of the immunochromatography assay, particularly an animal as well as a human, for example, blood, serum, blood plasma, spinal fluid, tear fluid, sweat, urine, feces, pus, nasal discharge, nasal swab, pharynx swab, nasal aspirate, or sputum, as well as an organ, a tissue, a mucous membrane, and skin, or swabs containing these, or an animal or plant itself or a dried form thereof. Examples of the test substance 71 include an antigen, an antibody, a protein, and a low-molecular-weight compound.

The photometry opening 18 is a rectangular opening for observing, from the outside, the assay region L1 or the like of the assay strip 15. The photometry opening 18 is formed between the sample dropping port 17 and the second pressing operation part 20.

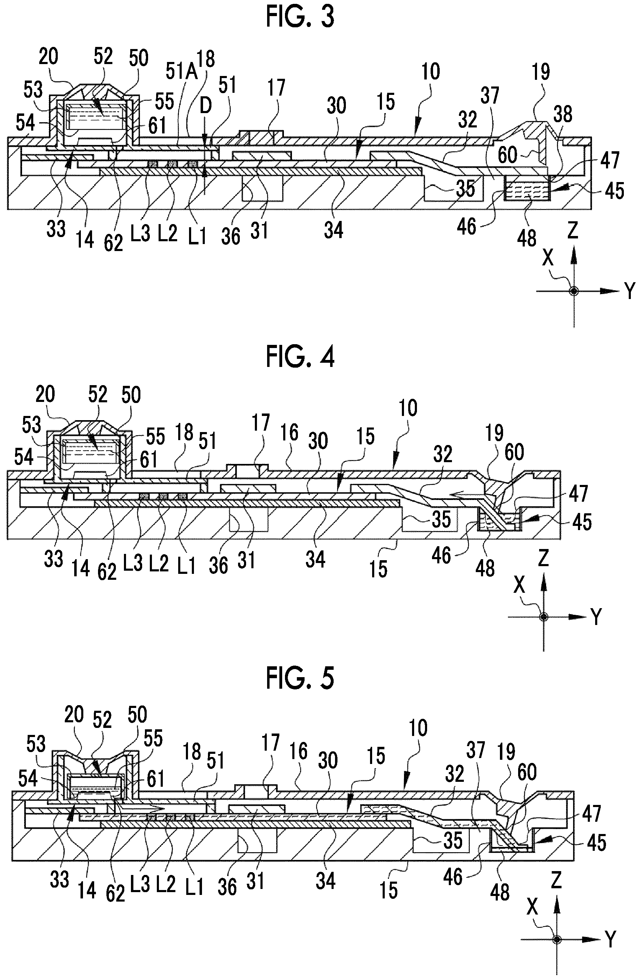

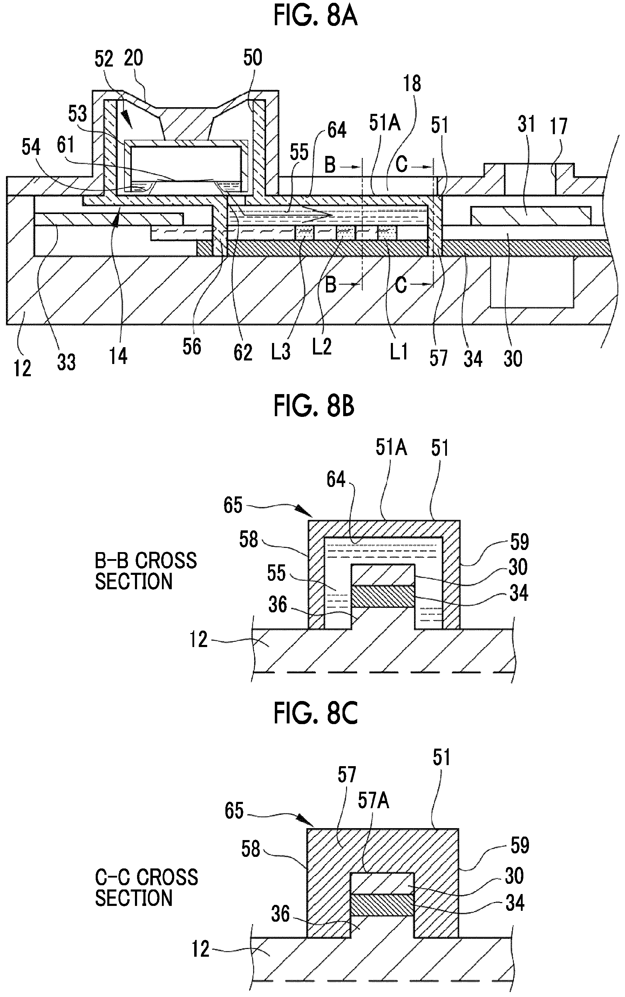

The first pressing operation part 19 is provided in one end part of the cover member 13 in the Y direction. The first pressing operation part 19 is subjected to a pressing operation by a user in a case where the washing solution 48 (see Fig. 4) is supplied to the assay strip 15. The second pressing operation part 20 is provided in the other end part of the cover member 13 in the Y direction, which is on a side opposite to the first pressing operation part 19. The second pressing operation part 20 is subjected to a pressing operation by a user in a case where the luminescent substrate solution 55 (see Fig. 8) is supplied to the assay strip 15.

As shown in Fig. 2, the assay strip 15 has an elongated rectangular plate shape as a whole and has a carrier 30, a label holding pad 31, a liquid feeding pad 32, an absorption pad 33, and a back pressure-sensitive adhesive sheet 34.

The carrier 30 is formed from a porous insoluble material such as a nitrocellulose membrane. The label holding pad 31 is attached to the position of the carrier 30 that faces the sample dropping port 17. The sample solution 70 dropped onto the sample dropping port 17 is spotted on the label holding pad 31. That is, the label holding pad 31 functions as a spotting region of the sample solution 70.

The sample solution 70 spotted on the label holding pad 31 permeates the carrier 30 and develops to one end side of the carrier 30 in the Y direction because of a capillary action. An assay region L1, a control region L2, and a color development region L3 are provided on one end side of the carrier 30 in the Y direction to which the sample solution 70 is developed. The assay region L1, the control region L2, and the color development region L3 are strip-shaped regions extending from one end to the other end of the carrier 30 in the X direction. In a case where a direction from the label holding pad 31 toward the assay region L1 and the like is defined as the development direction of the sample solution 70, the assay region L1 is positioned on the most upstream side in the development direction and the color development region L3 is positioned on the most downstream side in the development direction. The control region L2 is positioned between the assay region L1 and the color development region L3. In other words, the control region L2 is positioned on the downstream side of the assay region L1 and on the upstream side of the color development region L3. In Fig. 2, the assay region L1, the control region L2, and the color development region L3 are hatched, but the hatching is for convenience of description and does not indicate that each of the regions L1 to L3 develops color. The same applies to the following Figs. 3 to 5, 8, 13 to 15, 17, and the like.

The liquid feeding pad 32 is attached to one end of the carrier 30, which is on a side opposite to the other end of the carrier 30 having the assay region L1 or the like. The liquid feeding pad 32 is formed from a porous material similarly to the carrier 30 and the like, and it feeds the washing solution 48 to the carrier 30 by the capillary action.

The absorption pad 33 is attached to one end on a side opposite to the other end of the carrier 30 to which the label holding pad 31 is attached. The absorption pad 33 is formed from a porous material similarly to the carrier 30. The absorption pad 33 absorbs the sample solution 70, the washing solution 48, and the luminescent substrate solution 55, which are developed on the carrier 30.

The back pressure-sensitive adhesive sheet 34 is a base material of which the surface is a pressure-sensitive adhesive surface, and the carrier 30 is adhesively fixed thereto.

The back pressure-sensitive adhesive sheet 34 and then the assay strip 15 are placed on protruding support tables 35 and 36 which are formed in the accommodation space of the case main body 12. The support table 35 is provided in a central part of the case main body 12. The support table 36 is provided in one end part of the case main body 12 on the downstream side in the development direction of the sample solution 70. The support tables 35 and 36 are at the same height.

A protruding support table 37 is also formed in the other end part of the case main body 12 on the upstream side in the development direction of the sample solution 70. The support table 37 is at the same height as the support tables 35 and 36. The liquid feeding pad 32 is placed on the support table 37.

A first housing part 38 having a recessed shape is formed in the support table 37. The first housing part 38 is provided at a position where it faces one end of the liquid feeding pad 32, which is on a side opposite to the other end attached to the carrier 30. The washing solution holding part 45 is accommodated in the first housing part 38.

The washing solution holding part 45 is composed of a container 46 that has an opening on one surface and a film 47 that liquid-tightly covers the opening of the container 46. The container 46 is formed from, for example, a resin material. The washing solution 48 is stored in the inside of the container 46. The film 47 is, for example, an aluminum sheet, and it can be easily broken. The washing solution holding part 45 is accommodated in the first housing part 38 with the film 47 being faced upward so that the film 47 faces the other end of the liquid feeding pad 32.

The intermediate case member 14 is disposed on the upper portion of the support table 36. The intermediate case member 14 is formed from a transparent resin material, for example, an acrylic resin. The intermediate case member 14 is a member in which a second housing part 50 and a flow channel forming part 51 are integrally provided. The second housing part 50 is a box of which the upper surface is open, and the luminescent substrate solution holding part 52 is accommodated in the inside of the second housing part 50. The flow channel forming part 51 is a flat plate that extends from the bottom part of the second housing part 50 in the Y direction. The flow channel forming part 51 extends to the front of the label holding pad 31 and covers the upper part of the assay region L1, the control region L2, and the color development region L3 of the carrier 30. A spacing D is provided between the carrier 30 and the flow channel forming part 51 (see Fig. 3). The spacing D is, for example, in a range of 0.01 mm to 1 mm. Since the spacing D is provided between the carrier 30 and the flow channel forming part 51 in this way, a flow channel of the luminescent substrate solution 55 is ensured. Apart of the flow channel forming part 51 is exposed to the photometry opening 18. A portion of the flow channel forming part 51 that is exposed to the photometry opening 18 constitutes a photometric window part 51A. Since the flow channel forming part 51 is formed of a transparent resin material, photometry of light from the assay region L1 can be performed from the outside through the photometric window part 51A. The photometric window part 51A is exposed from the photometry opening 18.

The luminescent substrate solution holding part 52 is constituted by a container 53 that has an opening on one surface and a film 54 that liquid-tightly covers the opening of the container 53. The container 53 is formed from, for example, a resin material. It is noted that the luminescent substrate solution holding part 52 is preferably constituted by a light shielding member. This is because deterioration of the quality of the luminescent substrate 55A can be suppressed by accommodating the luminescent substrate 55A inside the light shielding member. The luminescent substrate solution 55 is stored in the inside of the container 53. The film 54 is, for example, a sealing film such as an aluminum sheet, and can be easily broken. The luminescent substrate solution holding part 52 is accommodated in the second housing part 50 with the film 54 being faced downward so that the film 54 faces the assay strip 15.

As shown in Fig. 3 as an example, a push-in protrusion member 60 is provided in the first pressing operation part 19. In addition, the luminescent substrate solution holding part 52 is attached to an inner surface of the second pressing operation part 20. A perforating member 61 and a supply port 62 are provided at the bottom part of the second housing part 50. The perforating member 61 has a pointed tip.

As shown in Fig. 4 as an example, in a case where the first pressing operation part 19 is subjected to a pressing operation, the push-in protrusion member 60 abuts on the other end of the liquid feeding pad 32 and pushes the other end of the liquid feeding pad 32 toward the film 47 of the washing solution holding part 45. The film 47 is broken by the pushing in of the other end of the liquid feeding pad 32 by the push-in protrusion member 60, and the other end of the liquid feeding pad 32 is dropped into the container 46 and immersed in the washing solution 48. The washing solution 48 is developed from the other end of the liquid feeding pad 32 toward the carrier 30 by the capillary action. The first pressing operation part 19 is maintained in a crushed state even after being crushed by the pressing operation. Therefore, the development of the washing solution 48 is continued until substantially the entire washing solution 48 is sucked up to the liquid feeding pad 32.

As shown in Fig. 5 as an example, in a case where the second pressing operation part 20 is subjected to a pressing operation, the second pressing operation part 20 is deformed inward of the case 11, and thereby, the luminescent substrate solution holding part 52 moves downward in the second accommodating part 50 and reaches the bottom part of the second accommodating part 50 having the perforating member 61. That is, the case 11 includes the second pressing operation part 20 as a variable part that can be changed to the inside in a portion covering the luminescent substrate solution holding part 52. Then, the luminescent substrate solution holding part 52 is moved toward the perforating member 61 by applying an external force to the second pressing operation part 20 which is a variable part. Then, the film 54 of the luminescent substrate solution holding part 52 is broken by the perforating member 61. The luminescent substrate solution 55 stored in the luminescent substrate solution holding part 52 flows from the broken portion of the film 54 to the supply port 62. Further, it flows through the flow channel ensured by the spacing D (see Fig. 3) and then is supplied to the carrier 30.

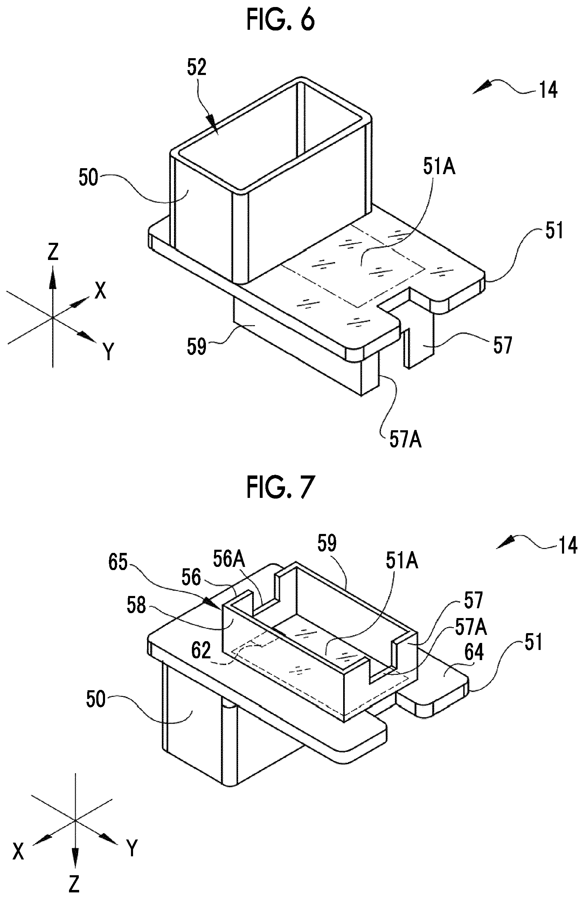

Fig. 6 is a perspective view of the intermediate case member 14, and Fig. 7 is a perspective view showing a surface side of the intermediate case member 14 facing the assay strip 15. Four wall portions 56, 57, 58, and 59 that are erected to surround the photometric window part 51A are provided on the facing surface 64 of the intermediate case member 14, which faces the assay strip 15 of the flow channel forming part 51. In a case where the intermediate case member 14 is disposed on the assay strip 15, cutouts 56A and 57A are provided at the center portions in the direction orthogonal to the longitudinal direction in the two wall portions 56 and 57 disposed along the direction orthogonal to the longitudinal direction of the assay strip 15. As shown in Fig. 6, the intermediate case member 14 is disposed on the assay strip 15 in an aspect of surrounding the assay region L1, the control region L2, and the color development region L3 of the assay strip 15 by the four wall portions 56 to 59 between the case main body 12 and the cover member 13.

Fig. 8 is an explanatory view of wall portions 56 to 59 of the intermediate case member 14. Fig. 8A is a side cross-sectional view of a part of the cartridge 10, Fig. 8B is a B-B cross-sectional view in Fig. 8A, and Fig. 8C is a C-C cross-sectional view in Fig. 8A. In a case where the assay region L1 is set as a reference position, the supply position side to which the luminescent substrate solution 55 is supplied is set as an upstream side, and the opposite side is set as a downstream side, the wall portion 56 is disposed on the upstream side of the flow channel, and the wall portion 57 is disposed on the downstream side of the flow channel. In the present example, since the development direction of the luminescent substrate solution 55 is opposite to the development direction of the sample solution 70, the upstream side and the downstream side in the development direction of the luminescent substrate solution 55 correspond to the downstream side and the upstream side in the development direction of the sample solution 70.

The two wall portions 56 and 57 extending in a direction crossing the flow channel are dam parts (hereinafter, also referred to as dam parts 56 and 57) that dam the flow of the luminescent substrate solution 55 supplied from the supply position to the flow channel. The remaining two wall portions 58 and 59 are connected to the dam parts 56 and 57, and are side walls (hereinafter, also referred to as side walls 58 and 59) disposed on the lateral of the flow path.

In a state where the assay strip 15 and the intermediate case member 14 are disposed in the case 11, a space surrounding the periphery of the assay region L1 is defined by the dam parts 56 and 57, the two side walls 58 and 59, and the facing surface 64. The luminescent substrate solution 55 supplied from the supply position flows along a flow channel constituted by a space formed between the assay strip 15 and the facing surface 64, but the flow to the upstream side with respect to the supply position is dammed by dam part 56 and the flow to the downstream side is dammed by the dam part 57. In addition, in a case where the luminescent substrate solution 55 overflows from the flow channel to the lateral of the flow channel by being dammed by the wall portions 56 and 57, the luminescent substrate solution 55 is dammed by the wall portions 58 and 59 on the lateral of the flow channel. As a result, the luminescent substrate solution 55 remains on the assay region L1. That is, the two dam parts 56 and 57, the two side walls 58 and 59, and the facing surface 64 constitute a retention part 65 that retains the luminescent substrate solution 55 on the assay region L1 (here, on a region including the assay region L1, the control region L2, and the color development region L3).

The luminescent substrate solution 55 flows through the flow channel, and a part of the luminescent substrate solution 55 permeates into the carrier 30 and is fed to the upstream side and the downstream side in the carrier 30 because of the capillary action. The luminescent substrate solution 55 that has remained on the assay region L1 is gradually absorbed by the carrier 30.

As shown in Fig. 9 as an example, the label holding pad 31 includes a labeled binding substance 77 labeled with the luminescent protein 76. The labeled binding substance 77 is a binding substance that specifically binds to the test substance. For example, in a case where the test substance 71 is an antigen, the labeled binding substance 77 is an antibody against the antigen, and in a case where the test substance 71 is an antibody, the labeled binding substance 77 is a secondary antibody that can bind to the antibody. In a case where the test substance 71 is a protein, a low-molecular-weight compound, or the like, the labeled binding substance 77 is an aptamer with respect to the protein, the low-molecular-weight compound, or the like.

The luminescent protein 76 is a protein that reacts with the luminescent substrate 55A to cause luminescence.

The binding substance for capturing 74 labeled with the fluorescent substance 73 is immobilized in the assay region L1. The binding substance for capturing 74 is a binding substance that specifically binds to the test substance 71. As a result, the test substance 71 is captured in the assay region L1. For example, in a case where the test substance 71 is an antigen, the binding substance for capturing 74 is an antibody against the antigen, and in a case where the test substance 71 is an antibody, the binding substance for capturing 74 is an antigen against the antibody. In a case where the test substance 71 is a protein, a low-molecular-weight compound, or the like, the binding substance for capturing 74 is an aptamer with respect to the protein, the low-molecular-weight compound, or the like.

The fluorescent substance 73 is a fluorescent dye, a fluorescent protein, or the like, which causes fluorescence to be generated in a case where excitation energy is applied.

The binding substance for capturing 74 and the labeled binding substance 77 may be the same or different from each other. For example, in a case where the test substance 71 is an influenza A type virus or a biomarker thereof, it is possible to use an anti-influenza A type monoclonal antibody (manufactured by Medix Biochemica Inc., product name: Anti-influenza A SPT N-5 7307) as the binding substance for capturing 74 and the labeled binding substance 77.

As the fluorescent substance 76 and the luminescent protein 73, those having an overlap between the wavelength range of the emission spectrum of the luminescent protein 76 and the wavelength range of the excitation spectrum of the fluorescent substance 73 are selected. Energy transfer can occur in a case where there is a wavelength range in which the emission spectrum of the luminescent protein 76 overlaps with the excitation spectrum of the fluorescent substance 73.

The luminescent protein 76 is, as an example, a luminescent protein that emits blue light. In this case, the fluorescent substance 73 can be allowed to be, as an example, a green fluorescent dye or a green fluorescent protein, which emits green fluorescence.

The luminescent substrate 55A reacts with the luminescent protein 76 and then is oxidized to generate energy. This causes an excited state, and luminescence occurs in a case where a transition to the ground state occurs. On the other hand, in a case where the fluorescent substance 73 and the luminescent protein 76 are positioned in a state of being close to each other, a bioluminescence resonance energy transfer (BRET) occurs in which the energy (hereinafter, referred to as luminescence energy E) generated by the reaction between the luminescent substrate 55A and the luminescent protein 76 is transferred to the fluorescent substance 73. As a result, fluorescence 78 is generated. That is, due to the luminescence energy E that occurs by the reaction between the luminescent substrate 55A and the luminescent protein 76, the fluorescent substance 73 is excited to cause fluorescence 78 to be generated from the fluorescent substance 73. In the assay using the cartridge 10 of the present embodiment, it is determined whether the sample is positive or negative by detecting the fluorescence 78 generated from the fluorescent substance 73 due to this BRET. The details of the assay procedure will be described later.

Specific examples of the combination of the luminescent protein 76, the fluorescent substance 73, and the luminescent substrate 55A include a combination of NanoLuc (registered trade name, manufactured by Promega Corporation) which is a blue luminescent protein, mNeongreen (manufactured by Allele Biotechnology and Pharmaceuticals, Inc.) which is a green fluorescent protein, and Furimazine (manufactured by Promega Corporation).

The control region L2 includes a control binding substance 75 labeled with a fluorescent substance 73. The control binding substance 75 specifically binds to the labeled binding substance 77. As a result, the luminescent protein 76 is captured in the control region L2. Some labeled binding substances 77 may not bind to the test substance 71. Such a labeled binding substance 77 reaches the control region L2 without being captured in the assay region L1 and then is captured in the control region L2. In a case where the luminescent protein 76 is captured via the labeled binding substance 77, the fluorescent substance 73 and the luminescent protein 76 are positioned close to each other in the control region L2. In a case where the luminescent substrate 55A is developed in this state, BRET occurs in the control region L2. The fluorescence generated from the fluorescent substance 73 by BRET makes it possible to confirm that the sample solution 70 has been sufficiently developed on the carrier 30 and the development of the sample solution 70 has been completed.

The control binding substance 75 may be the test substance 71 itself or may be a compound having a moiety that is recognized by the labeled binding substance 77. Examples of the compound having a moiety that is recognized by the labeled binding substance 77 include such a compound that is obtained by bonding a derivative of the test substance 71 to a protein. For example, in a case where the test substance 71 is an influenza A type virus or a biomarker thereof, it is possible to use, as the control binding substance 75, an anti-mouse IgG antibody (manufactured by Fujifilm Wako Pure Chemical Corporation, product name: anti-mouse IgG (H + L), rabbit F(ab')2, product code: 566-70621).

The color development region L3 contains a substance (not shown) of which the color development state changes by reacting with the washing solution 48. In a case where the color development region L3 develops or changes color by reacting with the washing solution 48, it is understood that the washing solution 48 is developed to the color development region L3, and the timing of starting the supply of the luminescent substrate solution 55 is reached. For example, in a case where a buffer solution containing a surfactant prepared to be acidic is used as the washing solution 48, the color development region L3 is preferably constituted by a color developing reagent immobilization line in which Bromocresol Green manufactured by FUJIFILM Wako Pure Chemical Corporation is immobilized in a strip shape. In this case, the color development region L3 is dark green before reacting with the washing solution 48, and changes to orange in a case where the washing solution 48 reaches.

The washing solution 48 is supplied to the assay region L1 after the sample is supplied to the assay region L1 and before the luminescent substrate 55Ais supplied to the assay region L1. The washing solution 48 is used to wash the labeled binding substance 77 that has non-specifically adsorbed in the assay region L1. Here, the "labeled binding substance 77 is non-specifically adsorbed" means that the labeled binding substance 77 is in a state of being adsorbed on the carrier without depending on the specific binding between the test substance 71 and the binding substance for capturing 74. Since the labeled binding substance 77 that has non-specifically adsorbed can be removed by the washing solution 48, the background can be reduced and the S/N can be improved.

The washing solution 48 is a liquid that can wash away the labeled binding substance 77 and the test substance 71 that have non-specifically adsorbed, and does not weaken the specific binding or cause a decrease in sensitivity of light detection in later steps. Examples of the washing solution 48 include a surfactant, a buffer solution containing a preservative, and the like.

The luminescent substrate solution 55 is a solution obtained by dissolving the above-described luminescent substrate 55A in a solvent. The luminescent substrate 55A is appropriately selected according to the luminescent protein 76. As the solvent, a liquid that can dissolve the luminescent substrate 55A, and does not weaken the specific binding or cause a decrease in sensitivity of photodetection in a subsequent step is used. Furthermore, it is preferable that the solvent has a function of washing away the labeled binding substance 77 and the test substance 71 that have non-specifically adsorbed. Examples of the solvent include an organic solvent, a mixed liquid of an organic solvent with water, and the like.

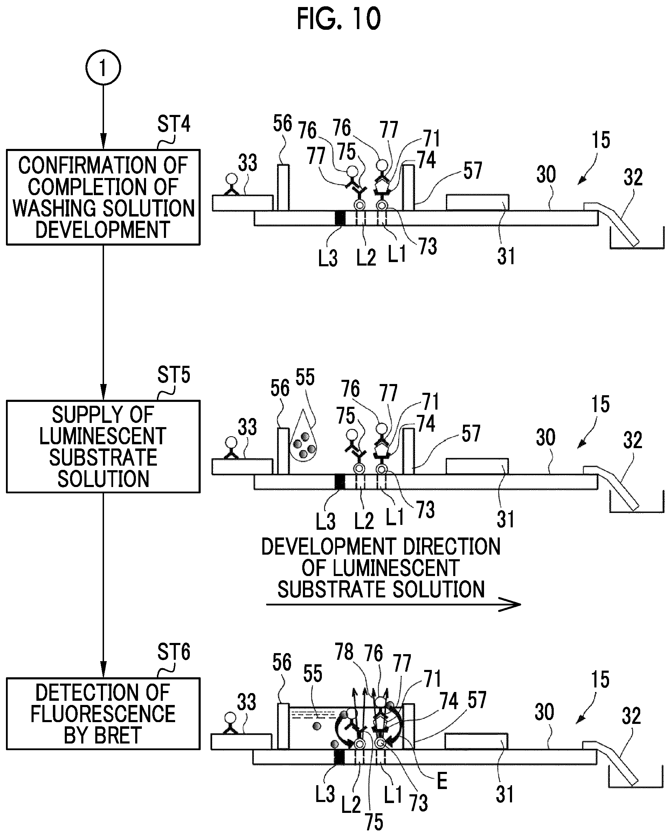

Hereinafter, the procedure of the immunochromatographic assay will be described with reference to Figs. 9 and 10. Here, a description will be made using, as an example, a case where a sample contains the test substance 71, that is, a case where the sample is positive. It is noted that in Figs. 9 and 10, the back pressure-sensitive adhesive sheet 34 is not illustrated in the drawing.

First, as shown in a step ST1, the sample solution 70 is spotted on the label holding pad 31. The test substance 71 in the sample solution 70, which has been spotted on the label holding pad 31, specifically binds to the labeled binding substance 77 of the label holding pad 31.

As shown in Step ST2, the sample solution 70 permeates from the label holding pad 31 to the carrier 30 and is developed on the assay region L1 side because of the capillary action. The test substance 71 that has reached the assay region L1 is captured by the binding substance for capturing 74. As a result, the luminescent protein 76 is captured by the binding substance for capturing 74 of the assay region L1 via the test substance 71 and the labeled binding substance 77 (see Step ST3). It is noted that the labeled binding substance 77 that is not captured in the assay region L1 is further developed up to the absorption pad 33. In addition, a part of the labeled binding substances 77 may not be developed to the absorption pad 33 and may be non-specifically adsorbed on the carrier 30.

Next, as shown in a Step ST3, the washing solution 48 is supplied. The washing solution 48 is supplied to the carrier 30 via the liquid feeding pad 32 and is developed on the assay region L1 side because of the phenomenon action. By this development of the washing solution 48, the labeled binding substance 77 that has non-specifically adsorbed on the carrier 30 is washed. In this case, the labeled binding substance 77 that has non-specifically adsorbed in the assay region L1 is allowed to flow to the absorption pad 33 together with the washing solution 48.

Thereafter, as shown in Step ST4, the washing solution 48 reaches the color development region L3, and a change occurs in the color development state of the color development region L3, thereby it is confirmed that the development of the washing solution 48 is completed.

After confirming the development completion of the washing solution 48, the luminescent substrate solution 55 is supplied as shown in Step ST5. The luminescent substrate solution 55 is supplied to the carrier 30 from one end on the opposite side of the label holding pad 31 with the assay region L1 interposed therebetween, and is developed on the assay region L1 side.

As shown in Step ST6, the luminescent substrate 55A contained in the luminescent substrate solution 55 that has reached the assay region L1 reacts with the luminescent protein 76 that has been captured in the assay region L1. Then, BRET in which the luminescence energy E generated by this reaction is transferred to the fluorescent substance 73 immobilized in the assay region L1 occurs, and the fluorescence 78 is generated from the luminescent protein 76. By detecting the fluorescence 78 from the assay region L1, it is possible to determine whether the sample is positive or negative.

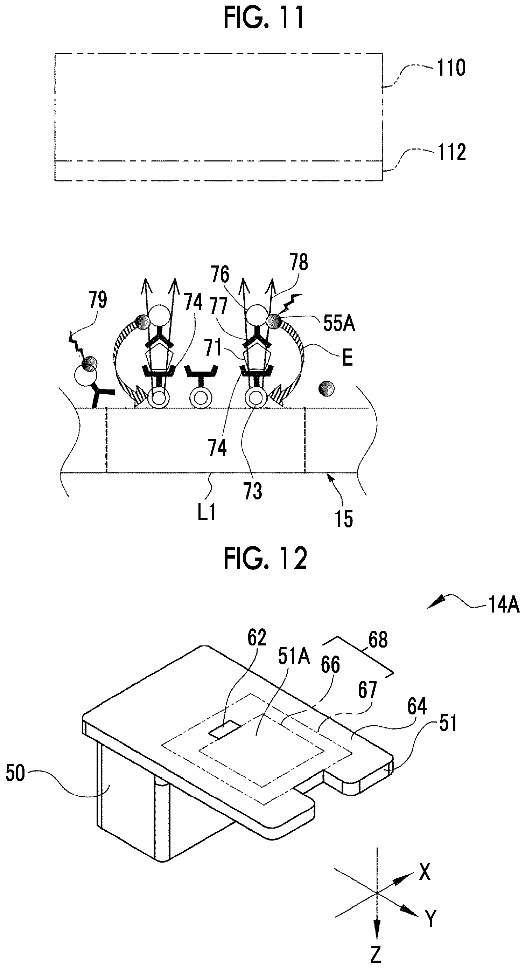

It is noted that the luminescence from the assay region L1 is detected by a photodetector 110 (see Fig. 11) of a detection device (not shown).

In a case where the sample is positive, as shown in Fig. 11, the test substance 71 is captured by the binding substance for capturing 74 of the assay region L1, and the luminescent protein 76 is captured via the test substance 71. In this state, in a case where the luminescent substrate 55A is supplied to the assay region L1, the luminescent substrate 55A reacts with the luminescent protein 76 to generate luminescence energy E, and the fluorescence 78 is generated from the fluorescent substance 73 due to BRET. In this way, in a case where the sample is positive, the fluorescence 78 due to the BRET is detected as light from the assay region L1.

It is noted that in a case where the sample is negative, since the test substance 71 is not contained in the sample, the test substance 71 is not captured by the binding substance for capturing 74 of the assay region L1, and as a result, the luminescent protein 76 is also not captured in the assay region L1. In this state, even in a case where the luminescent substrate 55A is supplied to the assay region L1, since the luminescent protein 76 is not present in the assay region L1, light is not detected from the assay region L1.

It is noted that in a case where the luminescent protein 76 and the fluorescent substance 73 are not close to each other, in the assay region L1, BRET does not occur, and the luminescent protein 76 and the luminescent substrate 55A may react with each other to generate light 79. The light 79 may serve as a background and hinders the detection of the fluorescence 78. As shown in Fig. 11, the optical filter 112 that attenuates the light 79 generated by the reaction between the luminescent protein 76 and the luminescent substrate 55A and allows the fluorescence 78 from the fluorescent substance 73 to pass is provided between the assay region L1 and the photodetector 110, thereby the incidence of the light 79 on the photodetector 110 can be suppressed. As the photodetector 110, an imaging element such as a charge coupled device (CCD) camera or a complementary metal oxide semiconductor (CMOS) camera can be used. A spectroscopic photodetector may be used as the photodetector 110. In a case where the photodetector 110 is a spectroscopic photodetector, the photodetector 110 can detect light for each wavelength, and thus the optical filter 112 may not be provided.

The present cartridge 10 includes a retention part 65 that retains the luminescent substrate solution 55 on the assay region L1 in a case where the luminescent substrate solution 55 is developed on the assay strip 15. As shown in Step ST6 of Fig. 10, the luminescent substrate solution 55 can be retained on the assay region L1 for a long time as compared with a case where the retention part 65 is not present. The chemiluminescence or the light due to BRET is not emitted in a case where the luminescent substrate is not present in the vicinity of the luminescent protein, and it takes a certain amount of time to reach the peak of luminescence after the luminescent substrate solution 55 is supplied to the assay strip 15. According to the present cartridge 10, since the luminescent substrate solution 55 can be retained on the assay region L1, the peak of luminescence can be increased, and the light detection can be performed with high accuracy.

The retention part 65 is provided inside the cartridge 10, and in a case where the luminescent substrate solution 55 is supplied to the assay strip 15, it is not necessary for the user to perform work for retaining the luminescent substrate solution 55 on the assay region L1. Therefore, according to the present cartridge 10, the assay by the chemiluminescence immunochromatographic method can be reliably and easily realized without burdening the user.

In the present example, the dam parts 56 and 57 and the two side walls 58 and 59 connected to the dam parts 56 and 57 are provided, and the two side walls 58 and 59 are connected to the facing surface 64. Then, the retention part 65 is constituted by the two dam parts 56 and 57, the two side walls 58 and 59, and the facing surface 64, and defines a space surrounding the assay region L1. The luminescent substrate solution 55 can be efficiently retained in this space.

The retention part 65 is not limited to the above-described configuration, and any configuration may be adopted as long as the luminescent substrate solution 55 can be retained for a longer time than in a case where the retention part 65 is not present on the assay region L1.

In the above-described embodiment, the wall portions 56 to 59 are erected on the facing surface 64 of the flow channel forming part 51, but a square tubular member including the four wall portions 56 to 59 may be provided as a separate member independent of the flow channel forming part 51 (that is, the intermediate case member 14).

In addition, as in the intermediate case member 14A of the modification example shown in Fig. 12, a retention part 68 may be constituted by a first region 66 and a second region 67 that are provided on the facing surface 64 of the flow channel forming part 51 of the intermediate case member 14A. The first region 66 is a region including a facing region facing the assay region L1. The second region 67 is a region surrounding the periphery of the first region 66. The second region 67 has a lower affinity for the luminescent substrate solution 55 than the first region 66.

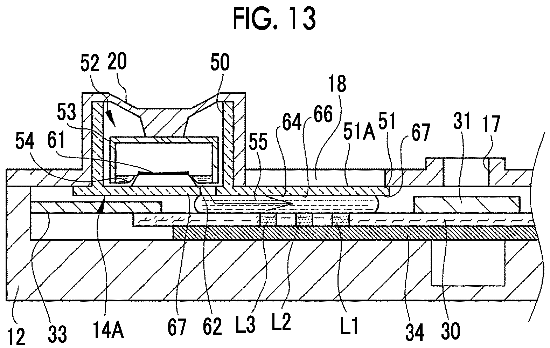

In the example shown in Fig. 12, the first region 66 is a region corresponding to the photometric window part 51A. Since the first region 66 has a higher affinity for the luminescent substrate solution 55 than the second region 67, the luminescent substrate solution 55 supplied to the flow channel can be retained in the first region 66 as shown in Fig. 13.

Examples of the method of making the affinity for the luminescent substrate solution 55 lower in the second region 67 than in the first region 66 include a method of performing a hydrophilization treatment or a hydrophobization treatment on the first region 66 and/or the second region 67.

In a case where the polarity of the luminescent substrate solution 55 is high, the affinity of the second region 67 for the luminescent substrate solution 55 can be made lower than that of the first region 66 by performing a hydrophilization treatment on the first region 66 to form a hydrophilized region and/or performing a hydrophobization treatment on the second region 67 to form a hydrophobized region. The first region 66 may be subjected to the hydrophilization treatment only, or the second region 67 may be subjected to the hydrophobization treatment only, but by subjecting the first region 66 to the hydrophilization treatment to form a hydrophilized region and subjecting the second region 67 to the hydrophobization treatment to form a hydrophobized region, the confinement effect of the highly polar luminescent substrate solution 55 in the first region 66 is high.

In a case where the polarity of the luminescent substrate solution 55 is low, the affinity of the second region 67 for the luminescent substrate solution 55 can be made lower than that of the first region 66 by performing a hydrophobization treatment on the first region 66 to form a hydrophobized region and/or performing a hydrophilization treatment on the second region 67 to form a hydrophilized region. In this case as well, the first region 66 may be subjected to the hydrophobization treatment only, or the second region 67 may be subjected to the hydrophilization treatment only, but by subjecting the first region 66 to the hydrophobization treatment to form a hydrophobized region and subjecting the second region 67 to the hydrophilization treatment to form a hydrophilized region, the confinement effect of the lowly polar luminescent substrate solution 55 in the first region 66 is high.

Examples of the hydrophobization treatment include a method of applying a silane coupling agent having a hydrophobic group to the surface of the facing surface. Examples of the hydrophobic group include an alkyl chain, a phenyl group, and the like.

Examples of the hydrophilization treatment include a method of applying a silane coupling agent having a hydrophilic group to the surface of the facing surface. Examples of the hydrophilic group include a hydroxyl group, a polyethylene glycol (PEG) chain, and the like. In addition, examples of the hydrophilization treatment also include a method of forming a hydroxyl group by an O₂ ashing treatment, a plasma treatment, or the like.

Even in a case where the retention part 68 formed by such a hydrophilization treatment or hydrophobization treatment is provided, the luminescent substrate solution 55 can be retained on the assay region L1 for a long time as compared with a case where the retention part 68 is not present. Therefore, it is possible to increase the luminescent peak of fluorescence due to chemiluminescence or BRET and to perform photodetection with high accuracy.

In addition, the cartridge 10 includes, in the cartridge 10, the luminescent substrate solution holding part 52 that encompasses the luminescent substrate solution 55 containing the luminescent substrate 55A. Since the luminescent substrate solution 55 is provided in the cartridge 10, the work of, for example, measuring the luminescent substrate solution 55 from a container such as an ampule or a vial and supplying the luminescent substrate solution 55 to the assay region L1, decreases. The present cartridge 10 includes the second pressing operation part 20, and thus the luminescent substrate solution 55 can be supplied to the assay strip 15 in a case where the second pressing operation part 20 is pushed down, whereby a simple assay can be realized.

Furthermore, the cartridge 10 includes the washing solution holding part 45 that encompasses the washing solution 48 in the cartridge 10. Since the washing solution 48 is provided in the cartridge 10, the work of, for example, measuring the washing solution 48 from a container such as an ampule or a vial and supplying the washing solution 48 to the assay region L1, decreases. The present cartridge 10 includes the first pressing operation part 19, and thus the washing solution 48 can be supplied to the assay strip 15 in a case where the first pressing operation part 19 is pushed down, whereby a simple assay can be realized.

In the technology of the present disclosure, the washing solution holding part 45 is not an essential component. However, since the labeled binding substance 77, the test substance 71, and the like that are non-specifically adsorbed can be washed away by being provided with the washing solution holding part 45 and developing the washing solution 48 after the sample solution 70 is developed on the assay region L1 of the assay strip 15 and before the luminescent substrate solution 55 is supplied, the background can be suppressed and the S/N in the case of light detection can be improved.

As described above, the first pressing operation part 19 and the second pressing operation part 20 of the cartridge 10 can be subjected to a pressing operation by a user. However, it is also possible to perform pressing operation in the assay apparatus including the first pressing operation part 19 and the second pressing operation part 20.

In the cartridge 10 of the above-described embodiment, the case 11 includes the second pressing operation part 20 as a variable part that is deformable inward in a portion covering the luminescent substrate solution holding part 52. Then, it is configured such that the luminescent substrate solution holding part 52 is moved toward the perforating member 61 by applying an external force to the second pressing operation part 20 which is a variable part. With this form, the second pressing operation part 20 is subjected to the pressing operation by the user, thereby the luminescent substrate solution 55 can be supplied to the assay strip 15. In addition, in the present example, the luminescent substrate solution holding part 52 is moved toward the perforating member 61, but the film 54 is only required to be perforated by the perforating member 61 by the relative movement of the luminescent substrate solution holding part 52 and the perforating member 61. Therefore, the perforating member 61 may be configured to move toward the luminescent substrate solution holding part 52 by an external force, or both the luminescent substrate solution holding part 52 and the perforating member 61 may be configured to move toward each other.

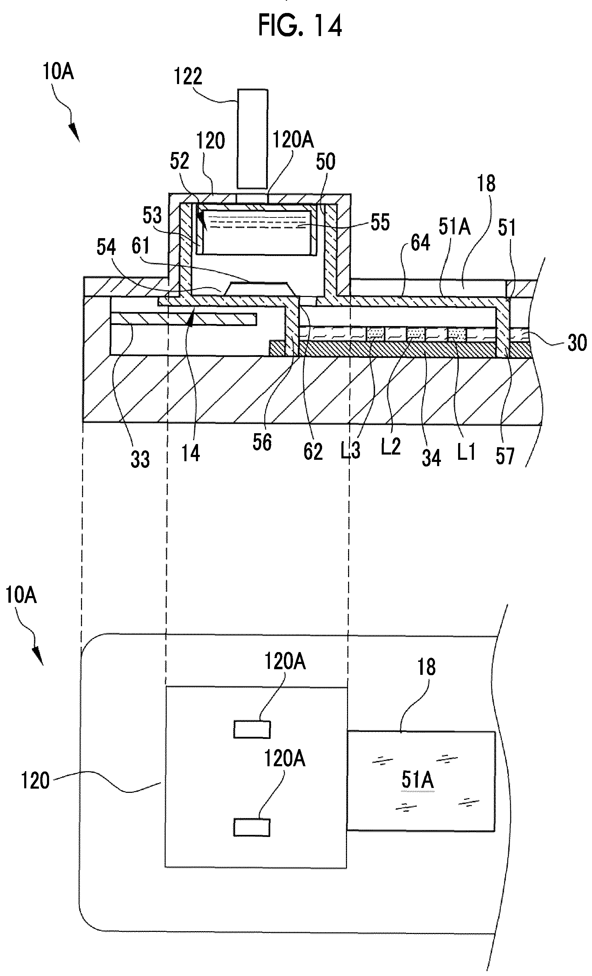

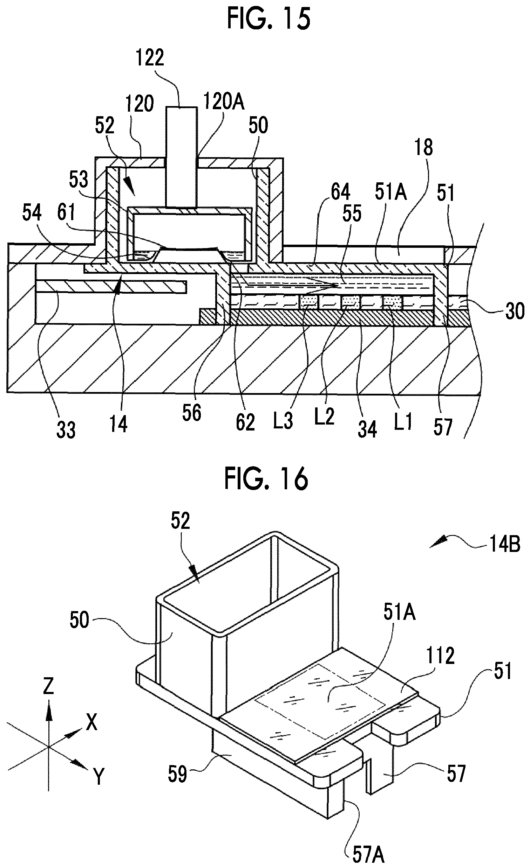

In addition, the case 11 is not limited to the above-described form. Fig. 14 shows a side cross-sectional view and a plan view of a part of a cartridge 10A of a modification example. In Fig. 14, the same components as those of the cartridge 10 shown in Figs. 1 to 8 are denoted by the same reference numerals. The same applies to Fig. 15. In the cartridge 10A of the modification example, the case 11A does not include a variable part that is deformable inward like the second pressing operation part 20. The case 11A includes a hole portion 120A (two hole portions 120A in the present example) on the upper surface of the protruding portion 120 that covers the luminescent substrate solution holding part 52. The luminescent substrate solution holding part 52 is disposed to be movable by a pressing member 122 that is inserted into the hole portion 120A.

In a case where the luminescent substrate solution 55 is supplied to the assay strip 15, as shown in Fig. 15, the pressing member 122 is inserted into the hole portion 120A, and the tip of the pressing member 122 is abutted on the luminescent substrate solution holding part 52 and pushed thereinto, thereby the luminescent substrate solution holding part 52 is moved toward the perforating member 61. The pressing member 122 may be a form of being inserted into the hole portion 120A by the user manually, or may be a form in which the cartridge 10A is loaded into the assay apparatus and the pressing member 122 provided in the assay apparatus is inserted into the hole portion 120A.

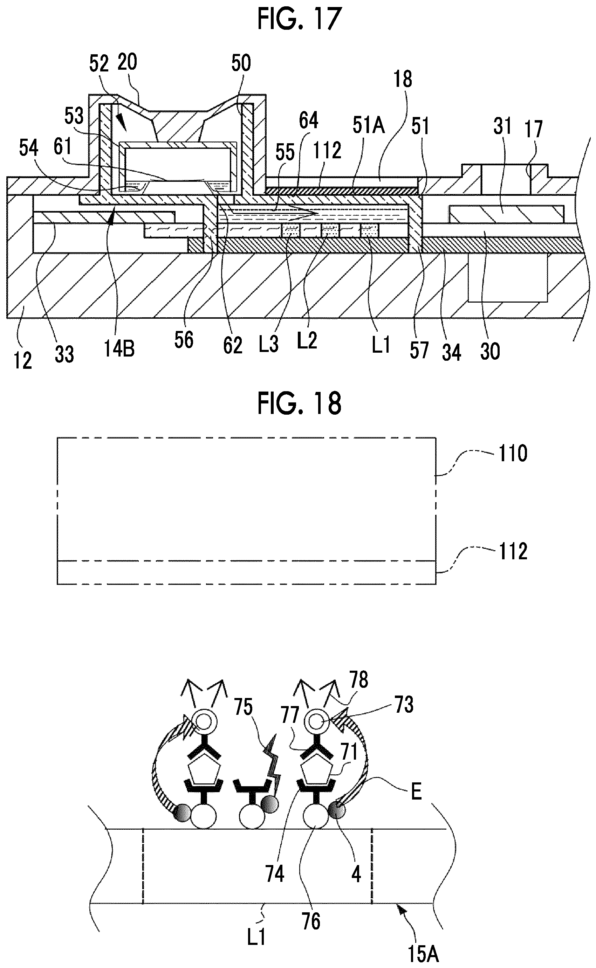

In the above-described embodiment, in a case of detecting the light from the assay region L1, to suppress the incidence of the light 79 of the chemiluminescence on the photodetector 110, the optical filter 112 is provided between the assay region L1 and the photodetector 110. The optical filter 112 may be provided in the cartridge 10. The intermediate case member 14B of the modification example shown in Fig. 16 includes an optical filter 112 in the photometric window part 51A of the flow channel forming part 51. Fig. 17 shows a side cross-sectional view of the cartridge in a case where the intermediate case member 14B shown in Fig. 16 is provided. As shown in Fig. 17, a photometric window part 51A exposed to the photometry opening 18 is covered with an optical filter 112. In the example shown in Fig. 16, the optical filter 112 is provided over the entire width of the flow channel forming part 51 including the photometric window part 51A, but the optical filter 112 is only required to be formed in at least the photometric window part 51A exposed to the photometry opening 18. In addition, as in the present example, the optical filter 112 may be provided on a surface of the photometric window part 51A that is on the outside of the case 11, or may be provided on the facing surface 64 that is the inner surface. The optical filter 112 attenuates the light 79 of the chemiluminescence and transmits the fluorescence 78. As described above, in a case where the cartridge 10 includes the optical filter 112, it is not necessary to include the optical filter 112 on the photodetector 110 side.

In the cartridge 10 of the above-described embodiment, the labeled binding substance 77 is labeled with the luminescent protein 76, and the binding substance for capturing 74 is labeled with the fluorescent substance 73. However, the labeled binding substance 77 may be labeled with the fluorescent substance 73 and the binding substance for capturing 74 may be labeled with the luminescent protein 76.

Fig. 18 is an explanatory view of light from the assay region L1 in a case where the labeled binding substance 77 is labeled with the fluorescent substance 73 and the binding substance for capturing 74 is labeled with the luminescent protein 76. In this case, the luminescent protein 76 is immobilized in the assay region L1 of the assay strip 15A. The test substance 71 in the sample solution 70 added dropwise from the sample dropping port 17 is bound to the labeled binding substance 77, and the fluorescent substance 73 is added via the labeled binding substance 77. Then, the test substance 71 is captured by the binding substance for capturing 74 of the assay region L1, and the fluorescent substance 73 is captured via the test substance 71. In this state, in a case where the luminescent substrate 55A is supplied to the assay region L1, the luminescent substrate 55A reacts with the luminescent protein 76 to generate luminescence energy E, and the fluorescence 78 is generated from the fluorescent substance 73 due to BRET. In addition, in the assay region L1, a binding substance for capturing 74 that has not captured the test substance 71 is also present. In a case where the luminescent protein 76 of the binding substance for capturing 74 that has not captured the fluorescent substance 73 reacts with the luminescent substrate 55A, BRET does not occur, and the light 79 of chemiluminescence is generated. That is, in a case where the sample is positive, the fluorescence 78 and the light 79 are generated from the assay region L1.

Since the fluorescence 78 and the light 79 have different peak wavelengths, only the fluorescence 78 can be detected by providing the optical filter 112 that attenuates the light 79 and transmits the fluorescence 78 between the photodetector 110 and the assay region L1.

It is noted that in a case where the sample is negative, since the test substance 71 is not contained in the sample, the test substance 71 is not captured by the binding substance for capturing 74 of the assay region L1, and as a result, the fluorescent substance 73 is also not captured in the assay region L1. In this state, in a case where the luminescent substrate 55A is supplied to the assay region L1, even in a case where the luminescent substrate 55A reacts with the luminescent protein 76 to generate luminescence energy, BRET does not occur, thereby fluorescence is not generated and the light 79 of chemiluminescence is generated. That is, in a case where the sample is negative, only the light 79 is generated from the assay region L1. Since the light 79 is attenuated by the optical filter 112, the light detector 110 hardly detects the light from the assay region L1.

As described above, even in a case where the labeled binding substance 77 is labeled with the fluorescent substance 73 and the binding substance for capturing 74 is labeled with the luminescent protein 76, the sample can be determined to be negative or positive by detecting the fluorescence 78 due to BRET in the assay region L1. In addition, also in this case, since the cartridge 10 includes the retention part 65, the luminescent substrate solution 55 can be retained on the assay region L1, and the luminescent protein 76 and the luminescent substrate 55A can be sufficiently reacted with each other in the assay region L1 to generate light (light energy).

The technique of the present disclosure can also be applied to a cartridge for performing a chemiluminescence immunochromatographic method without using BRET.

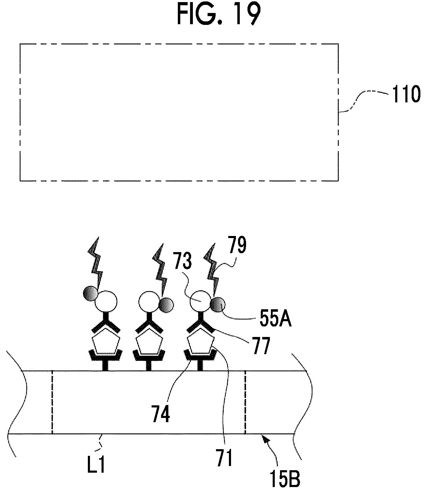

In the chemiluminescence immunochromatographic method without using BRET, as shown in Fig. 19, the binding substance for capturing 74 that is not labeled is immobilized in the assay region L1 of the assay strip 15B, and the luminescent protein 76 is used as a label of the labeled binding substance 77. In a case where the sample is positive, the labeled binding substance 77 labeled with the luminescent protein 76 is captured via the test substance 71. The luminescent substrate 55A is supplied to the assay region L1 in this state, and thus the luminescent substrate 55A reacts with the luminescent protein 76 to emit light. In this case, the luminescent substrate 55A and the luminescent protein 76 react with each other to generate light 79 due to chemiluminescence. Then, the light 79 is detected as the light from the assay region L1. In a case where the sample is negative, since the test substance 71 to be captured by the binding substance for capturing 74 is not present, the luminescent protein 76 is not captured in the assay region L1. Therefore, even in a case where the luminescent substrate 55Ais supplied to the assay region L1, the light 79 due to chemiluminescence is not generated. That is, in a case of negative, light is not generated from the assay region L1.

Even in the case of the chemiluminescence immunochromatographic method without using BRET, to accurately detect the light 79 generated by the reaction between the luminescent substrate 55A and the luminescent protein 76, it is necessary to react the luminescent substrate 55A with the luminescent protein 76 for a certain period of time. Therefore, by providing the retention part 65 and retaining the luminescent substrate solution 55 on the assay region L1, the effect of improving the detection accuracy can be obtained in the same manner as in the case of BRET.

Provided that in a case where the BRET is not used and a case where the labeled binding substance 77 that has non-specifically adsorbed in the assay region L1 remains, the light generated by the reaction between the luminescent protein 76 which is a label of the labeled binding substance 77, and the luminescent substrate 55A is a factor that increases the background. In a case where BRET is used, fluorescence is detected as a signal. Therefore, even in a case where the labeled binding substance 77 that has non-specifically adsorbed is present in the assay region L1, an increase in background due to the labeled binding substance 77 that has non-specifically adsorbed can be suppressed.

More specifically, in the immunochromatographic method using BRET, the luminescence energy E generated by the reaction between the luminescent substrate 55A and the fluorescent substance 73 is transferred to the luminescent protein 76 in a state where the labeled binding substance 77 is captured via the test substance 71, and the fluorescence 78 is generated from the luminescent protein 76. A phenomenon called BRET, in which this energy is transferred, occurs in a case of a state where the fluorescent substance 73 and the luminescent protein 76 are close to each other (for example, in a state where they are positioned to each other at a distance of about 10 nm or less). As a result, in a case where the labeled binding substance 77 is not captured by the binding substance for capturing 74, BRET does not occur since the fluorescent substance 73 and the luminescent protein 76 are not close to each other. Therefore, even in a case where the labeled binding substance 77 is non-specifically adsorbed in the peripheral region of the assay region L1, the luminescent protein 76 labeled to this labeled binding substance 77 that has non-specifically adsorbed is not excited, and fluorescence is not generated. As a result, even in a case where the labeled binding substance 77 that has non-specifically adsorbed is generated in the peripheral region of the assay region L1, the background does not increase, which makes it possible to carry out measurement with a high S/N ratio. That is, the cartridge 10 according to the present embodiment, which enables determination by a measurement using BRET, makes it possible to suppress the background as compared with the above-described chemiluminescence immunochromatographic method in which this BRET is not used, and thus the measurement accuracy can be improved.

The following appendixes are further disclosed with respect to the above embodiment.

### (Appendix 1)

An immunochromatographic cartridge comprising:
an assay strip having an assay region in which a sample is developed and a test substance contained in the sample is captured;
a binding substance that specifically binds to the test substance and is labeled with a luminescent protein;
a luminescent substrate solution holding part that accommodates a luminescent substrate solution containing a luminescent substrate that is supplied to the assay region and that reacts with the luminescent protein to generate light; and
a case that accommodates the assay strip and the luminescent substrate solution holding part and that includes a photometric window part through which light from the assay region is measurable from outside,
wherein the case further includes a retention part that retains the luminescent substrate solution on the assay region in a case where the luminescent substrate solution is developed on the assay strip.

### (Appendix 2)

The immunochromatographic cartridge according to Appendix 1,
wherein in the case, the luminescent substrate solution holding part is disposed to be capable of supplying the luminescent substrate solution to the assay strip from above the assay strip, and
the case includes a flow channel forming part that is disposed to face a surface of the assay strip in the assay region, in which a part of the flow channel forming part constitutes the photometric window part, and
the luminescent substrate solution is supplied to the assay region through between the assay strip and a facing surface of the flow channel forming part facing the assay strip as a flow channel.

### (Appendix 3)

The immunochromatographic cartridge according to Appendix 2,
wherein in the flow channel, in a case where the assay region is set as a reference position, a side of a supply position where the luminescent substrate solution is supplied is set as an upstream side, and an opposite side is set as a downstream side,
the immunochromatographic cartridge has two dam parts that are provided at two positions of the upstream side and the downstream side of the flow channel and that dam a flow of the luminescent substrate solution supplied to the flow channel from the supply position, and
   two side walls that are connected to the two dam parts and that are disposed on a lateral of the flow channel,
the two dam parts and the two side walls are connected to the facing surface, and
the retention part is constituted by the two dam parts, the two side walls, and the facing surface, and defines a space surrounding a periphery of the assay region.

### (Appendix 4)

The immunochromatographic cartridge according to Appendix 2,
wherein the facing surface of the flow channel has a first region including a facing region facing the assay region and a second region that surrounds a periphery of the first region and that has an affinity for the luminescent substrate solution lower than that of the first region, and
the retention part is constituted by the first region and the second region.

### (Appendix 5)

The immunochromatographic cartridge according to Appendix 4,
wherein the first region is a hydrophilized region subjected to a hydrophilization treatment, and the second region is a hydrophobized region subjected to a hydrophobization treatment.

### (Appendix 6)

The immunochromatographic cartridge according to any one of Appendixes 1 to 5,
wherein at least a part of the luminescent substrate solution holding part is constituted by a film,
the case further includes a perforating member that is disposed to face the film of the luminescent substrate solution holding part and that perforates the film, and
in the case, the film is perforated by the perforating member by relative movement of the luminescent substrate solution holding part and the perforating member, to supply the luminescent substrate solution onto the assay strip.

### (Appendix 7)

The immunochromatographic cartridge according to Appendix 6,
wherein the case includes a variable part that is deformable inward in a portion covering the luminescent substrate solution holding part, and that moves the luminescent substrate solution holding part toward the perforating member by applying an external force.

### (Appendix 8)

The immunochromatographic cartridge according to Appendix 6 or 7,
wherein the case includes a hole portion in a portion covering the luminescent substrate solution holding part, and
the luminescent substrate solution holding part is movable toward the perforating member by a pressing member to be inserted into the hole portion.

### (Appendix 9)

The immunochromatographic cartridge according to any one of Appendixes 1 to 8,
wherein the luminescent substrate solution holding part is constituted by a light shielding member.

### (Appendix 10)

The immunochromatographic cartridge according to any one of Appendixes 1 to 9,
wherein in the case, a washing solution holding part that accommodates a washing solution which is supplied to the assay region and by which a binding substance non-specifically adsorbed to the assay region is washed is further provided.

### (Appendix 11)

The immunochromatographic cartridge according to any one of Appendixes 1 to 10,
wherein the assay strip includes a binding substance for capturing that is immobilized on the assay region, that is labeled with a fluorescent substance, and that specifically binds to the test substance, and the assay strip includes a labeled binding substance that is disposed on an upstream side of the assay region in a case of developing the sample and that is supplied to the assay region, as the binding substance, and
in a case where the luminescent substrate is supplied to the assay region in a state where the labeled binding substance is captured by the binding substance for capturing that is immobilized on the assay region, through the test substance, a bioluminescence resonance energy transfer occurs in which energy of light generated by a reaction between the luminescent protein and the luminescent substrate is transferred to the fluorescent substance to generate fluorescence.

### (Appendix 12)

The immunochromatographic cartridge according to any one of Appendixes 1 to 10,
wherein the assay strip includes the binding substance as a binding substance for capturing that is immobilized in the assay region, and the assay strip further includes a labeled binding substance that is disposed on an upstream side of the assay region in a case where the sample is developed, that specifically binds to the test substance, that is labeled with a fluorescent substance, and that is supplied to the assay region, and
in a case where the luminescent substrate is supplied to the assay region in a state where the labeled binding substance is captured by the binding substance for capturing that is immobilized in the assay region, through the test substance, a bioluminescence resonance energy transfer occurs in which energy of light generated by a reaction between the luminescent protein and the luminescent substrate is transferred to the fluorescent substance to generate fluorescence.

### (Appendix 13)

The immunochromatographic cartridge according to Appendix 11 or 12,
wherein the case includes an optical filter that attenuates light generated by the reaction between the luminescent substrate solution and the luminescent protein and that allows transmission of the fluorescence through the photometric window part.

It is noted that the disclosure of JP2022-153102A filed on September 26, 2022, is incorporated in the present specification in its entirety by reference. All documents, patent applications, and technical standards disclosed in the present specification are incorporated in the present specification by reference to the same extent as those in a case where each of the documents, patent applications, and technical standards are specifically and individually indicated to be incorporated by reference.

## Claims

1. An immunochromatographic cartridge comprising:
an assay strip having an assay region in which a sample is developed and a test substance contained in the sample is captured;
a binding substance that specifically binds to the test substance and is labeled with a luminescent protein;
a luminescent substrate solution holding part that accommodates a luminescent substrate solution containing a luminescent substrate that is supplied to the assay region and that reacts with the luminescent protein to generate light; and
a case that accommodates the assay strip and the luminescent substrate solution holding part and that includes a photometric window part through which light from the assay region is measurable from outside,
wherein the case further includes a retention part that retains the luminescent substrate solution on the assay region in a case where the luminescent substrate solution is developed on the assay strip.

2. The immunochromatographic cartridge according to claim 1,
wherein in the case, the luminescent substrate solution holding part is disposed to be capable of supplying the luminescent substrate solution to the assay strip from above the assay strip, and
the case includes a flow channel forming part that is disposed to face a surface of the assay strip in the assay region, in which a part of the flow channel forming part constitutes the photometric window part, and
the luminescent substrate solution is supplied to the assay region through between the assay strip and a facing surface of the flow channel forming part facing the assay strip as a flow channel.

3. The immunochromatographic cartridge according to claim 2,
wherein in the flow channel, in a case where the assay region is set as a reference position, a side of a supply position where the luminescent substrate solution is supplied is set as an upstream side, and an opposite side is set as a downstream side,
the immunochromatographic cartridge has two dam parts that are provided at two positions of the upstream side and the downstream side of the flow channel and that dam a flow of the luminescent substrate solution supplied to the flow channel from the supply position, and
two side walls that are connected to the two dam parts and that are disposed on a lateral of the flow channel,
the two dam parts and the two side walls are connected to the facing surface, and
the retention part is constituted by the two dam parts, the two side walls, and the facing surface, and defines a space surrounding a periphery of the assay region.

4. The immunochromatographic cartridge according to claim 2,
wherein the facing surface of the flow channel has a first region including a facing region facing the assay region and a second region that surrounds a periphery of the first region and that has an affinity for the luminescent substrate solution lower than that of the first region, and
the retention part is constituted by the first region and the second region.

5. The immunochromatographic cartridge according to claim 4,
wherein the first region is a hydrophilized region subjected to a hydrophilization treatment, and the second region is a hydrophobized region subjected to a hydrophobization treatment.

6. The immunochromatographic cartridge according to any one of claims 1 to 5,
wherein at least a part of the luminescent substrate solution holding part is constituted by a film,
the case further includes a perforating member that is disposed to face the film of the luminescent substrate solution holding part and that perforates the film, and
in the case, the film is perforated by the perforating member by relative movement of the luminescent substrate solution holding part and the perforating member, to supply the luminescent substrate solution onto the assay strip.

7. The immunochromatographic cartridge according to claim 6,
wherein the case includes a variable part that is deformable inward in a portion covering the luminescent substrate solution holding part, and that moves the luminescent substrate solution holding part toward the perforating member by applying an external force.

8. The immunochromatographic cartridge according to claim 6,
wherein the case includes a hole portion in a portion covering the luminescent substrate solution holding part, and
the luminescent substrate solution holding part is movable toward the perforating member by a pressing member to be inserted into the hole portion.

9. The immunochromatographic cartridge according to claim 1,
wherein the luminescent substrate solution holding part is constituted by a light shielding member.

10. The immunochromatographic cartridge according to claim 1,
wherein in the case, a washing solution holding part that accommodates a washing solution which is supplied to the assay region and by which a binding substance non-specifically adsorbed to the assay region is washed is further provided.

11. The immunochromatographic cartridge according to claim 1,
wherein the assay strip includes a binding substance for capturing that is immobilized on the assay region, that is labeled with a fluorescent substance, and that specifically binds to the test substance, and the assay strip includes a labeled binding substance that is disposed on an upstream side of the assay region in a case of developing the sample and that is supplied to the assay region, as the binding substance, and
in a case where the luminescent substrate is supplied to the assay region in a state where the labeled binding substance is captured by the binding substance for capturing that is immobilized on the assay region, through the test substance, a bioluminescence resonance energy transfer occurs in which energy of light generated by a reaction between the luminescent protein and the luminescent substrate is transferred to the fluorescent substance to generate fluorescence.

12. The immunochromatographic cartridge according to claim 1,
wherein the assay strip includes the binding substance as a binding substance for capturing that is immobilized in the assay region, and the assay strip further includes a labeled binding substance that is disposed on an upstream side of the assay region in a case where the sample is developed, that specifically binds to the test substance, that is labeled with a fluorescent substance, and that is supplied to the assay region, and
in a case where the luminescent substrate is supplied to the assay region in a state where the labeled binding substance is captured by the binding substance for capturing that is immobilized in the assay region, through the test substance, a bioluminescence resonance energy transfer occurs in which energy of light generated by a reaction between the luminescent protein and the luminescent substrate is transferred to the fluorescent substance to generate fluorescence.

13. The immunochromatographic cartridge according to claim 11 or 12,
wherein the case includes an optical filter that attenuates light generated by the reaction between the luminescent substrate solution and the luminescent protein and that allows transmission of the fluorescence through the photometric window part.
